# EUROPÄISCHE PATENTSCHRIFT

(11) **EP 2 922 947 B1**
(45) Veröffentlichungstag und Bekanntmachung des Hinweises auf die Patenterteilung: **11.01.2017**
(21) Anmeldenummer: 13788750.1
(22) Anmeldetag: 07.11.2013
(51) Int. Cl.: C07B 59/00, B01L 1/02, G01N 33/58, C12M 1/00

(54) **VERFAHREN UND VORRICHTUNG ZUR ISOTOPENMARKIERUNG**
METHOD AND APPARATUS FOR ISOTOPE MARKING
PROCÉDÉ ET DISPOSITIF DE MARQUAGE ISOTOPE

(30) Priorität: 21.11.2012 US 201261728817 P; 21.11.2012 EP 12193566
(43) Veröffentlichungstag der Anmeldung: 30.09.2015
(73) Patentinhaber: BASF Plant Science Company GmbH, 67056 Ludwigshafen (DE)
(72) Erfinder: FUCHS, Regine, 10243 Berlin (DE); WOIWODE, Olaf, 14552 Michendorf (DE); PETER, Enrico, 13189 Berlin (DE); SCHÖN, Hardy, 13156 Berlin (DE); WITTMANN, Christoph, 38304 Wolfenbüttel (DE); RASCH, Detlev, 38124 Braunschweig (DE); BECKERS, Veronique, 38102 Braunschweig (DE); DERSCH, Lisa, 38106 Braunschweig (DE)
(74) Vertreter: BASF IP Association
(86) Internationale Anmeldenummer: PCT/EP2013/073245
(87) Internationale Veröffentlichungsnummer: WO 2014/079696

(56) Entgegenhaltungen:
- EP-A1- 2 518 137
- US-A- 5 324 636
- US-A- 5 352 414
- US-A1- 2010 136 675
- US-B1- 6 200 362

## Beschreibung

### GEBIET DER ERFINDUNG

Die vorliegende Erfindung betrifft eine Vorrichtung (Isotopenmarkierungskammer) zur Markierung von Organismen, vorzugsweise Pflanzen und Tiere, mit Isotopen und ein Markierungsverfahren mittels Verwendung der erfindungsgemäßen Vorrichtung.

### HINTERGRUND DER ERFINDUNG

Metabolische Flussanalysen (MFA) gewinnen zunehmend an Beachtung als zentraler Bestandteil der modernen Systembiologie. Anhand MFA werden die Bewegungsraten von Stoffwechselprodukten innerhalb komplexer intrazellulärer Netzwerke quantifiziert. Im Zusammenspiel mit Proteom-, Transkriptom- sowie Genomanalysen lassen sich mittels MFA genetische und umweltbedingte Einflüsse auf das Wachstum von Pflanzen und anderen Organismen darstellen.

Bei der Quantifizierung von Stoffwechselwegen über MFA hat sich die Markierung von Verbindungen, welche von der Pflanze aufgenommen werden, als zweckmäßig erwiesen (Szyperski (1998) 13C-NMR, MS and metabolic flux balancing in biotechnology research. Quart. Riev. Biophys. 31:41-106). In Frage kommen dabei Radioisotope, wie beispielsweise ¹⁴C ³H, oder ³²P, oder Stabilisotope, wie beispielsweise ²H, ¹³C, ¹⁸O, ¹⁵N. Die von der Pflanze aufgenommenen markierten Verbindungen wie z.B. markiertes Kohlendioxid, durchlaufen intrazelluläre Stoffwechselwege, wodurch die markierten Isotope in die interessierenden intrazellulären Metabolite oder Endprodukte des Metabolismus (zB Protein, Stärke, Lipid, Zellwand) verteilt und eingebaut werden. Die Markierung in Metaboliten oder Endprodukten des Metabolismus lassen sich anschließend über Massenspektrometrie oder Kernmagnetresonanz nachweisen. Die typischen Markierungsmuster von Zwischenstufen des zentralen Stoffwechsels oder seinen Endprodukten bilden somit einen "isotopen Fingerabdruck", mittels dessen die tatsächliche Flussverteilung berechnet werden kann.

Die experimentelle Bestimmung systemischer Stoffwechselflüsse unter Verwendung von ¹³C-Isotopen wird bereits seit langem zur Untersuchung verschiedener Organismen, wie z.B. Penicillium chrysogenum (Christensen and Nielsen, 2000), Escherichia coli (Fischer and Sauer, 2003; Zhao et al., 2004), diverse Hefen (Blank et al., 2005), Bacillus subtilis (Sauer et al., 1997), Corynebacterium glutamicum (Marx et al., 1996), Synechocystis (Yang et al., 2002), Methylobacterium extorquens (Van Dien et al., 2003), Soja-Embryos (Sriram et al., 2004) und Raps-Embryos (Schwender et al., 2004) eingesetzt
Im Gegensatz zu herkömmlichen ¹³C-Markierungsmethoden werden bei der isotopen, nichtstationären metabolischen Flussanalyse ("INST ¹³C-MFA" für *isotopic non-stationary meta-bolic flux analysis),* auch dynamische metabolische Flussanalyse genannt, von den Markierungsmustern Zeitprofile erstellt. Dabei wird die transiente Markierungsinformation der Metabolite zur Bestimmung der in vivo Flüsse verwendet (Übersicht siehe Nöh und Wiechert, 2011, Appl. Microbiol. Biotechnol., 91, Seiten 1247-1265), was mit stationären Verfahren, bei denen lediglich die Anreicherung des ¹³C nach Sättigung gemessen wird, nicht möglich ist. Das dynamische Verfahren erlaubt die metabolische Flussanalyse von photoautotrophen Systemen unter physiologischen Bedingungen und Verwendung von CO₂. Diese Analyse ist mit stationären Methoden nicht möglich, weshalb zur metabolischen Flussanalyse von Pflanzen in der Regel in-vitro Organkulturen herangezogen werden. In-vitro Organkulturen sind hoch artifizielle Systeme, daher ist die Analyse einer intakten Pflanze unter physiologischen Bedingungen vorteilhaft.

Verfahren und Vorrichtungen, die sich gegebenenfalls zur Isotopenmarkierung eignen, sind aus dem Stand der Technik bekannt. Beispielsweise beschreibt die DE1949001 eine Vorrichtung zur Regelung der Luftfeuchte in einer Pflanzenwuchskammer. Ein Mangel dieser Art Anordnung besteht in der Notwendigkeit einer künstlichen Lichtquelle aufgrund der Verwendung von nicht-transparentem Material für die Gehäusewände. Desweiteren fehlt es der in DE1949001 offenbarten Vorrichtung an einem Material zum Absorbieren von Kohlendioxid bevor die Markierung vorgenommen wird, sowie an geeigneten Vorrichtungen zur Zufuhr von CO₂.

US5352414 beschreibt ebenso eine Vorrichtung zur Markierung von Organismen, wobei eine Schleuse versehene Markierungskammer nicht vorgesehen ist.

Eine Vorrichtung zur Regelung und Bestimmung des Kohlendioxidgehaltes einer Wachstumskammer mittels geregelter Zu- und Ableitung von Kohlendioxid sowie eine Vorrichtung zur Absorption von Kohlendioxid sind beschrieben in DE 1773320; allerdings handelt es sich bei der in DE 1773320 beschriebenen Kammer um ein System zur Erfassung des Gesamt-Kohlendioxidumsatzes von Pflanzen (oder anderen Organismen wie Flechten) - ein Eingriff im Sinne einer kontinuierlichen Probennahme, wie für die Analyse metabolischer Flüsse benötigt und durch die erfindungsgemäß mit einer Schleuse versehene Markierungskammer ermöglicht, ist nicht vorgesehen.

Der technologische Hintergrund der vorliegenden Erfindung wird außerdem in US3673733 beschrieben, wobei mittels der in US3673733 beschriebenen Vorrichtung ebenfalls kein vorheriges Entfernen von nicht markiertem Kohlendioxid möglich ist.

US5341595 offenbart eine Kammer zur Analyse des Pflanzenwachstums, wobei mittels der in US5341595 beschriebenen Vorrichtung ebenfalls kein vorheriges Entfernen von nicht markiertem Kohlendioxid möglich ist. Darüber hinaus fehlt es der in US5341595 offenbarten Vorrichtung an einem System zur gleichmäßigen Belüftung.

Chen et al. (Proteome Science 2011, 9:9 http://www.proteomesci.com/content/9/1/9) offenbart eine geschlossene Pflanzenwachstumskammer, mittels derer Feuchtigkeit, Druck, Temperatur, und ¹³CO₂ Konzentration kontrolliert und konstant gehalten werden kann. Im Gegensatz zur vorliegenden Erfindung weist die in Chen et al. beschriebene Kammer allerdings keine Schleuse auf, die es ermöglichen würde, bei einer dynamischen MFA kontinuierlich Proben zu entnehmen, ohne dass ein Gasaustausch zwischen dem Kammerinneren und der äußeren Umgebung stattfindet.

Aus diesem Grund werden die Pflanzen bei Chen et al. mehrere Wochen in reiner ¹³CO2-Atmosphäre gehalten, wodurch sie nahezu vollständig mit ¹³CO2 markiert sind. Anschließend wird die Kammer geöffnet und stossartig gelüftet, worauf die zügige Probennahme erfolgt. Es wird also nicht, wie in der erfindungsgemäßen Kammer möglich, eine Anreicherung von ¹³C in den Pflanzen / Metaboliten gemessen, sondern eine Abreicherung des ¹³C, bzw. eine Anreicherung des ¹²C aus der umgebenden Atmosphäre. Die von Chen et al. beschriebene Methode ist somit wesentlich kostenaufwendiger, da eine ungleich höhere Menge ¹³CO2 benötigt wird.

Außerdem stellt das in Chen et al. beschriebene Verfahren eine weitaus größere Abweichung von natürlichen / physiologischen Bedingungen dar, als die lediglich kurze Exposition der zu markierenden Pflanzen mit ¹³CO2 im erfindungsgemäßen Verfahren, da die Pflanzen über einen längeren Zeitraum in einem abgeschlossenen System verbleiben, in dem eine künstliche Atmosphäre herrscht. Ein Unterschied zwischen ¹²CO₂ und ¹³CO₂ in der photosynthetischen Verwertung durch Pflanzen ist bekannt.

Die GMS Gaswechsel-Messsysteme GmbH Berlin bietet unter dem Produktnamen "BioBox" zur ¹³CO₂-Markierung von Pflanzen grundsätzlich geeignete Pflanzwachstumskammern kommerziell an, wobei ebenfalls eine künstliche Beleuchtung zur Anwendung kommt, und lediglich die kostenaufwendige Variante der Markierung bis zur ¹³CO₂-Sättigung mit anschliessender Messung der ¹³CO₂-Abnahme in den verschiedenen Metaboliten möglich ist. Auch hier müssen die zu markierenden Pflanzen über einen längeren Zeitraum in künstlicher Atmosphäre und unter künstlichen Beleuchtungsbedingungen verbleiben.

Für einzellige Organismen oder Flüssigkulturen von Pflanzenzellen ist die direkte Messung der ¹³C-Inkorporation im Sinne des INST-C-MFA Verfahrens aus dem Stand der Technik bekannt.

So zeigen beispielsweise Young et al. (2011, Metabolic Engineering 13, Seiten 656-665) eine Kohlenstoff-Flusskarte des einzelligen Cyanobakteriums Synechocystis, wobei die intrazellulären ¹³C-Verteilungen zur Berechnung metabolischer Flüsse unter photoautotrophen Bedingungen verwendet wurden. Die hierfür notwendige hohe Frequenz der Probennahme läßt sich bei der Verwendung von Flüssigkulturen beispielsweise durch die Entnahme eines Probevolumens durch einen Absperrhahn realisieren.

Eine Vorrichtung zur schnellen Probennahme und somit Messung der ¹³CO₂-Inkorporation höherer Pflanzen, welche einen wesentlich effizienteren, präziseren und naturnäheren Versuchsaufbau ermöglichen würde, ist aus dem Stand der Technik nicht bekannt.

Die Durchführung solcher Messungen, insbesondere an ganzen Erntepflanzen wie Mais, Reis, Soja oder Raps, ist demnach bislang nicht möglich.

Aufgabe der vorliegenden Erfindung war es daher, eine Markierungskammer für Pflanzen bereitzustellen, mittels derer zuverlässige dynamische metabolische Flussanalysen in Pflanzen durchgeführt werden können, ohne dass ein unerwünschter Gasaustausch zwischen dem Kammerinneren und Umgebung die Messergebnisse beeinflusst. Mit den Systemen aus dem Stand der Technik ist keine kontinuierliche Probennahme ohne nachhaltige Störung des Kammerklimas möglich. Darüber hinaus entspricht die in den bekannten Systemen erzielte ¹³CO₂-Sättigung über längeren Zeitraum nicht dem natürlichen Zustand. Mit der erfindungsgemäßen Vorrichtung wird außerdem weniger ¹³CO₂ verbraucht (und somit geringerer Kostenaufwand betrieben) als bei der in Chen et al. beschriebenen inversen Methode.

Gelöst wurde diese Aufgabe durch den Gegenstand der vorliegenden Erfindung, betreffend eine Isotopenmarkierungskammer zum Markieren von Stoffwechselprodukten in einem Organismus, vorzugsweise einer Pflanze, umfassend eine Reaktorkammer**(1)**, und eine Luftregulationskammer(2), dadurch gekennzeichnet, dass die Reaktorkammer**(1)** folgende Komponenten umfasst:
optional einen Gehäuserahmen **(3),** Gehäusewände **(4),** mindestens ein Injektionsventil **(5),** wobei mindestens eine Gehäusewand **(4)** vollständig und/oder teilweise öffenbar ist und wobei mindestens eine Gehäusewand eine Schleuse **(6)** aufweist,
und weiterhin dadurch gekennzeichnet, dass die Luftregulationskammer **(2)** folgende Komponenten umfasst:
   eine Temperaturregelungseinheit **(7),** eine Luftbefeuchtungseinheit **(8),** und eine Gasabsorptionseinheit **(9).**

Die Temperaturregelungseinheit, die Luftbefeuchtungseinheit und die Gasabsorptionseinheit sind jeweils über Kanäle und/oder Schläuche mit der oberen Reaktorkammer verbunden, und bilden somit voneinander unabhängige Kreisläufe und Luft-/Gasaustausche mit der Reaktorkammer.

Die Reaktorkammer(oberes Abteil: Gehäusewände und oberer Teil des Rahmens) ist als Modul gefertigt, also variierbar, nur die Luftregulationskammer (unteres Abteil) ist permanent mit Technik versehen, so dass Reaktor- bzw. Pflanzkammern verschiedener Größe mit der Luftregulationskammer kombiniert werden können.

### BESCHREIBUNG DER ZEICHNUNG

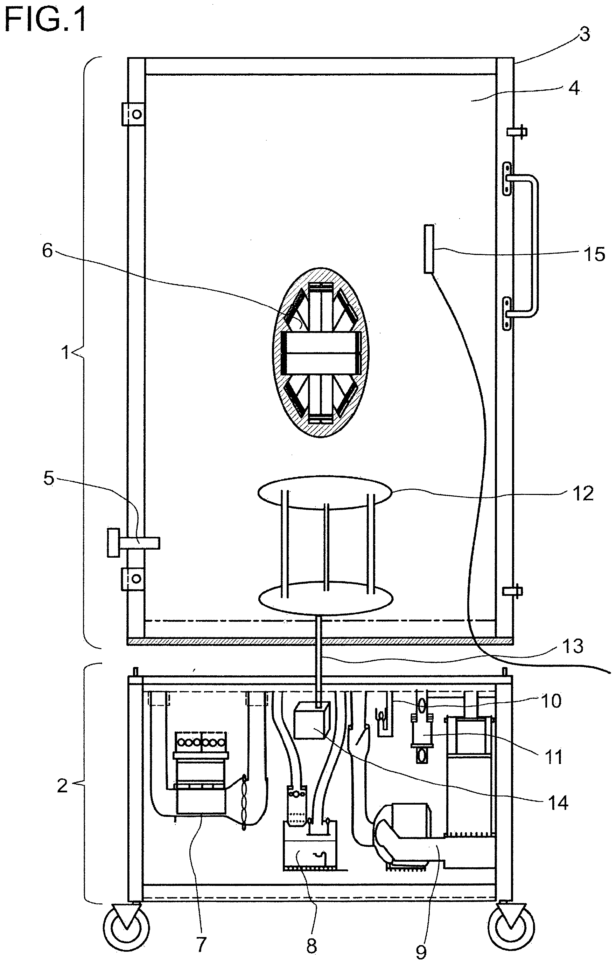

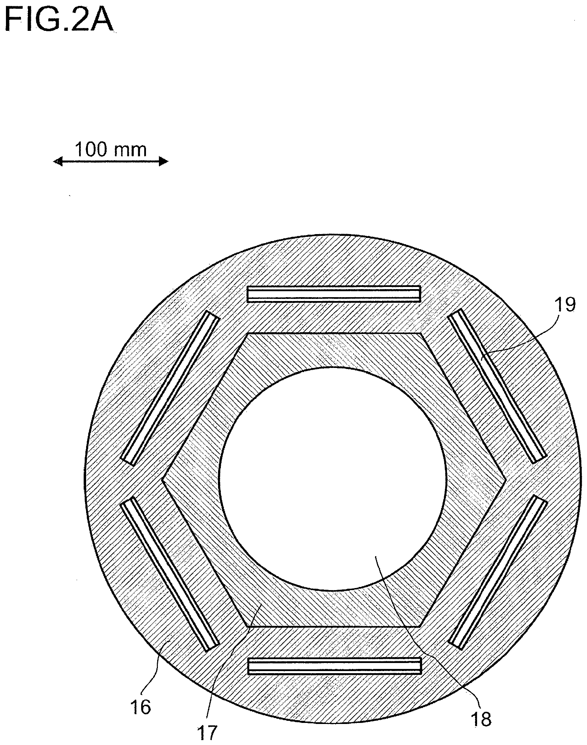

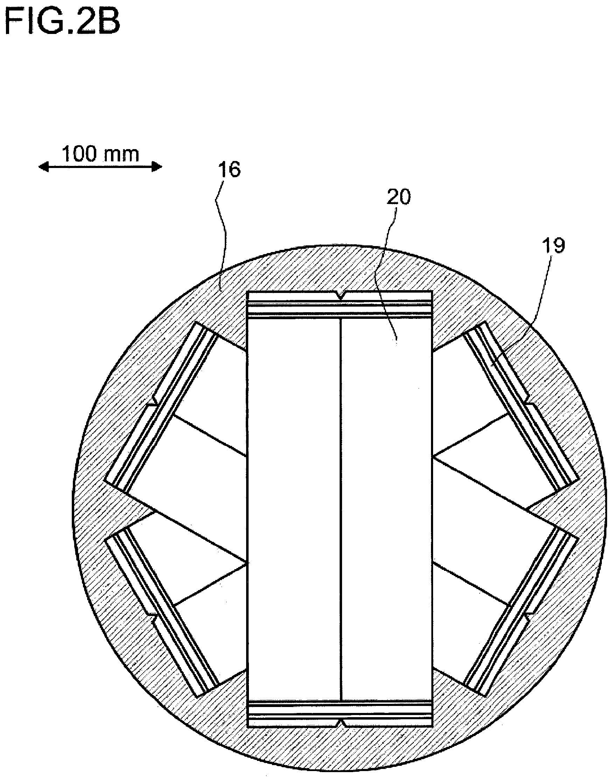

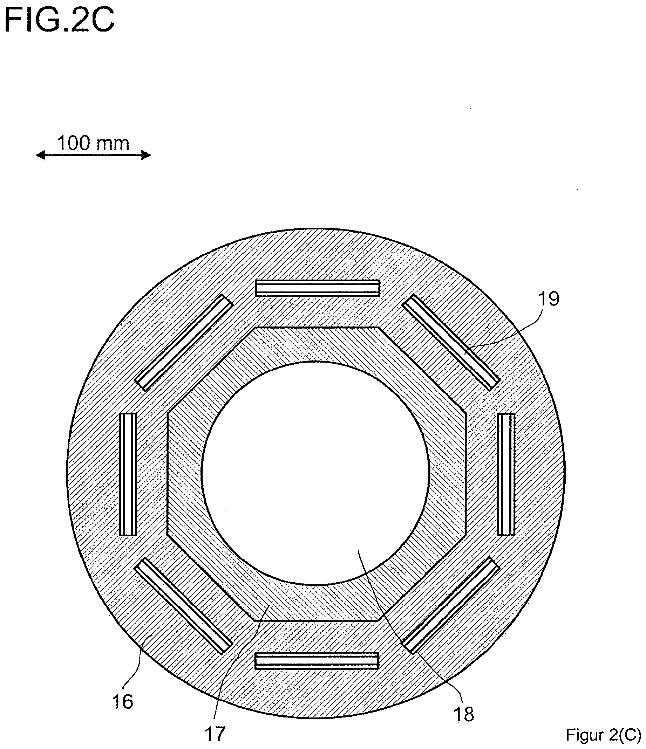

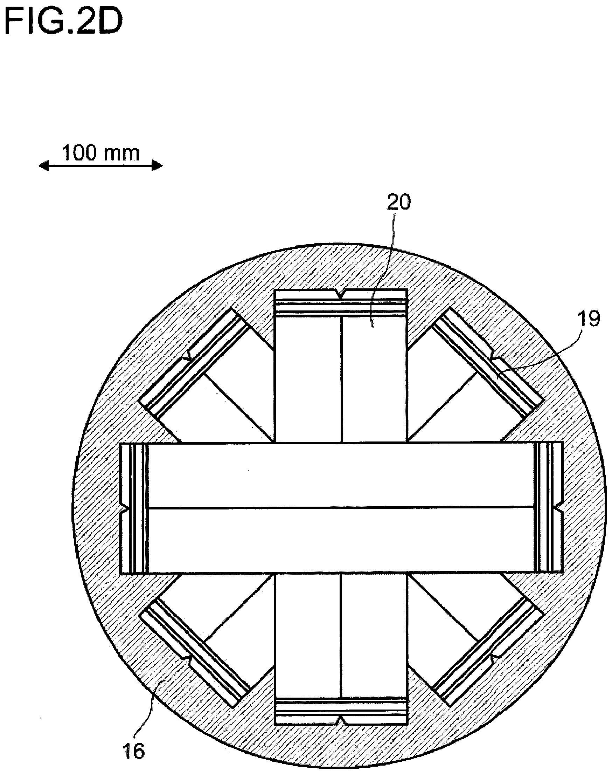

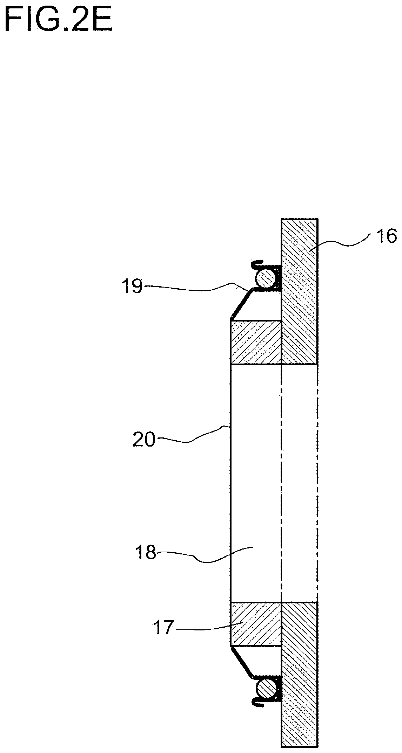

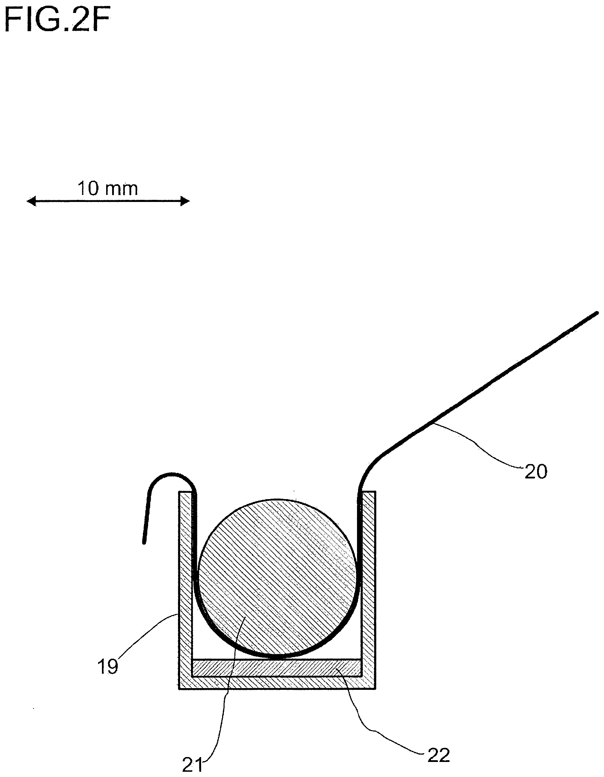

**Abb.** 1 zeigt eine Gesamtansicht der erfindungsgemäßen Markierungskammer wobei die Ziffern folgende Bedeutung haben:
   **(1)** Reaktorkammer
   **(2)** Luftregulationskammer
   **(3)** Rahmen
   **(4)** Gehäusewand
   **(5)** Injektionsventil
   **(6)** Schleuse
   **(7)** Temperaturregelungseinheit
   **(8)** Luftbefeuchtungseinheit
   **(9)** Gasabsortionseinheit
   **(10)** Überdruckventil
   **(11)** Unterdruckventil
   **(12)** Ablagetisch
   **(13)** Antriebsachse für Ablagetisch
   **(14)** Motor für Ablagetisch
   **(15)** CO₂-Messsonde
**Abb.** 2 zeigt eine erfindungsgemäße Schleuse, wobei die Ziffern folgende Bedeutung haben:
   (A) Aufbau der Kammerschleuse für sechs Latexbänder (offen, ohne Bänder): (16) Basisplatte; (17) Sechseckige Platte zum Auflegen der Latexbänder; (18) Durchgreiföffnung; (19) U-Profil;
   (B) Kammerschleuse mit sechs Latexbändern: (16) Basisplatte; (19) U-Profil; (20) Latexband;
   (C) Aufbau der Kammerschleuse für acht Latexbänder (offen, ohne Bänder): (16) Basisplatte; (17) Achteckige Platte zum Auflegen der Latexbänder; (18) Durchgreiföffnung; (19) U-Profil;
   (D) Kammerschleuse mit acht Latexbändern: (16) Basisplatte; (19) U-Profil; (20) Latexband;
   (E) Klemmmechanismus der Latexbänder: (16) Basisplatte; (17) Sechseckige Platte zum Auflegen der Latexbänder; (18) Durchgreiföffnung; (19) U-Profil; (20) Latexband
   (F) Klemmmechanismus Detailansicht: (19) U-Profil; (20) Latexband; (21) Rundstab; (22) Moosgummistreifen
**Abb.** 3 zeigt eine schematische Darstellung der Temperaturregelungseinheit mit Belüftungsvorrichtung, wobei die Ziffern folgende Bedeutung haben:
   (23) Luft / Auslassrohr; (24) Luft / Ansaugrohr; (25) Lüfter/Ventilator; (26) Peltierelemente für Heizung/Kühlung; (27) flexibles Alu-Lüftungsrohr; (28) Aluminium Rippenkühlkörper
**Abb.** 4 zeigt eine schematische Darstellung der Luftbefeuchtungsvorrichtung, wobei die Ziffern folgende Bedeutung haben:
   (25) Lüfter/Ventilator; (29) Luftansaugung; (30) Luftaustritt; (31) Wassergefäß mit Wasser gefüllt; (32) Ultraschall-Vernebler; (33) Spritzschutzplatte; (34) Flexibler Kunststoffschlauch
**Abb.** 5 zeigt die Gasabsorptionsvorrichtung (A) und die Druckausgleichsvorrichtung (B), wobei die Ziffern folgende Bedeutung haben:
   (A): (35) Ansaugöffnung; (36) Ausblasöffnung; (37) Rückschlagklappe; (38) Absorbermaterial; (39) Feinstaubfilter; (40) Pumpe; (41) Siebplatte
   (B): (10) Überdruckventil; (11) Unterdruckventil; (38) Absorbermaterial, (39) Feinstaubfilter; (42) Kugelventile
**Abb.** 6 zeigt das Ergebnis einer LC-MS/MS Messung am Beispiel eines zentralen Eintrittsmetaboliten des Kohlendioxid-Stoffwechsels, 3-Phosphoglycerat. Dargestellt sind die relativen Häufigkeiten der verschiedenen Massenisotopomere, wie sie durch die zunehmende Inkorporation von ¹³C beeinflußt werden:
   M0: ¹²C-Massenisotopomer des Analyten
   M1: einfach ¹³C-gelabeltes Massenisotopomer des Analyten (M1 = M0+1)
   M2: zweifach ¹³C-gelabeltes Massenisotopomer des Analyten (M2 = M0+2)
   M3: dreifach ¹³C-gelabeltes Massenisotopomer des Analyten (M3 = M0+3)

### BESCHREIBUNG DER BEVORZUGTEN AUSFÜHRUNGSBEISPIELE

### (1) Die erfindungsgemäße Isotopenmarkierungskammer

Es wird auf die Zeichnungen Bezug genommen, in denen gleiche Teile mit gleichen Ziffern bezeichnet sind.

Mit Bezug zuerst auf die **Abb.** 1 (dynamische Isotopenmarkierungskammer) umfaßt die erfindungsgemäße Kammer eine Reaktorkammer **(1),** und eine Luftregulationskammer **(2),** dadurch gekennzeichnet, dass die Reaktorkammer **(1)** folgende Komponenten umfasst: optional einen Gehäuserahmen **(3),** Gehäusewände **(4),** mindestens ein Injektionsventil **(5),** wobei mindestens eine Gehäusewand **(4)** vollständig und/oder teilweise öffenbar ist und wobei mindestens eine Gehäusewand eine Schleuse **(6)** aufweist,
und weiterhin dadurch gekennzeichnet, dass die Luftregulationskammer **(2)** folgende Komponenten umfasst:
eine Temperaturregelungseinheit **(7),** eine Luftbefeuchtungseinheit **(8),** und eine Gasabsorptionseinheit **(9).**

In der Kammer befindet sich typischerweise eine Ablagevorrichtung **(12),** vorzugsweise ein Tisch, auf dem die zu analysierenden Pflanzen platziert werden

In einer bevorzugten Ausführungsform besteht der Tisch aus Kunststoff, besonders bevorzugt aus Polyvinylchlorid, und ist in Form einer drehbaren Platte ausgeführt, welche durch einen von außen schaltbaren Getriebemotor **(14)** rotiert werden kann, um die Pflanzen innerhalb der Kammer in Reichweite der Schleuse zu positionieren, wodurch die mögliche Frequenz der Probennahme zusätzlich erhöht wird. In einer bevorzugten Ausführungsform kann sich der Motor, durch Verwendung einer die Kammerwand durchdringenden und zur Verhinderung unerwünschten Gasaustausches mit Radial-Wellendichtringen (sog. "Simmerringe") versehenen Achse, außerhalb der Pflanzenkammer befinden.

Die Größe der Kammer sollte so gewählt sein, dass sie sich zum Markieren von Pflanzen verschiedener Größe eignet. Bevorzugt ist die Größe der Kammer so gewählt, dass sie sich zur Markierung verschiedener Entwicklungsstadien von Erntepflanzen wie beispielsweise Mais, Soja, Reis, Raps, Baumwolle, Weizen, Roggen, Gerste, Triticale, Hirse, Hopfen, Kartoffel, Tabak, Tomate, Aubergine, Pfeffer, Öllein, Flachs, Sonnenblume, Erbse, sowie verschiedenen Büschen wie Kaffee, Kakao, Tee, Gräsern und Modellpflanzen wie Arabidopsis thaliana, eignet. Hierbei kann es sich bei den zu markierenden Pflanzen sowohl um Wildformen, als auch verschiedene Kultivare, Hybridsorten oder transgene Pflanzen verschiedener Entwicklungsstadien handeln.

In einer bevorzugten Ausführungsform ist die Markierungskammer modular aufgebaut aus einer Luftregulationskammer (als unterem Teil der Kammer) sowie einer Reaktorkammer (als oberem Teil der Kammer), wobei die Luftregulationskammer prinzipiell mit Reaktorkammern verschiedener Größen und Beschaffenheiten kombiniert werden kann, um Markierungsexperimente mit verschiedenen Pflanzen, Entwicklungsstadien und Rahmenbedingungen wie Anzahl der Pflanzen, Beleuchtungsbedingungen, Einwirkung von Pflanzenschutzmitteln oder Stressfaktoren wie Hitze, Kälte, Trockenheit, Salz etc. zu ermöglichen.

In einer bevorzugten Ausführungsform ist die Kammer zusammengesetzt aus Reaktor- und Luftregulationskammer 150-200cm hoch, 50-90cm breit, und 50-90cm tief, und weist ein Gesamtvolumen von 600-1000L auf. Die Reaktorkammer alleine ist vorzugsweise 80-130cm hoch, und weist ein Gesamtvolumen von 450-800L auf

In einer besonders bevorzugten Ausführungsform ist die Reaktorkammer 75 cm breit, 75 cm tief und 110 cm hoch und weist ein Volumen von rund 560L auf.

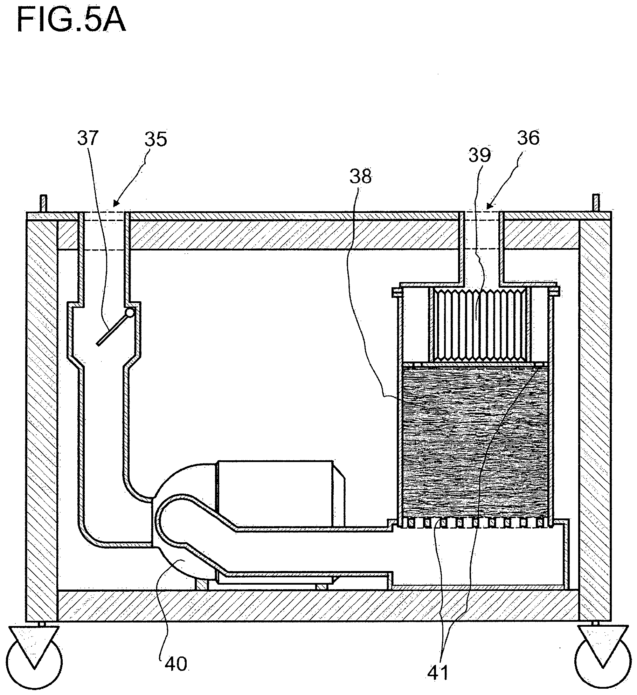

Die Gehäusewand **(4)** weist ein Injektionsventil **(5)** auf, das für ¹³CO₂ Injektionen geeignet ist. An einer anderen oder derselben Gehäusewand **(4)** kann sich ein Ausströmungskanal befinden (siehe Nr 35 in Abb 5 (A)), der mit der Gasabsorptionseinheit verbunden ist, und nicht-markiertes Kohlendioxid dorthin abführt. Das Injektionsventil eignet sich außerdem zum Einleiten von Schadstoffgasen, Herbiziden, weiteren, gasförmigen Isotopenmarkern, stabile oder radioaktiv markierte Gase (Sauerstoff, CO, Wasserdampf, Stickstoff)

Bei der mindestens einen vollständig und/oder teilweise öffenbaren Gehäusewand kann es sich um einen Deckel an der Oberseite der Reaktorkammer oder um eine Tür an einer der Seiten der Reaktorkammer handeln. Vorzugsweise ist die vollständig und/oder teilweise öffenbare Gehäusewand eine Tür. "Vollständig öffenbar" bedeutet, dass die ganze Gehäusewand als Tür angebracht ist; "teilweise öffenbar" bedeutet, dass sich in einer ansonsten geschlossenen Gehäusewand eine kleinere Öffnung in Form einer Tür befindet.

Eine Schleuse **(6)** in einer der Gehäusewände ermöglicht die kontinuierliche Probenentnahme zur Erstellung einer dynamischen Messreihe, weil damit ein Eindringen von nichtmarkiertem Kohlendioxid während der Messreihe verhindert wird. Die Schleuse befindet sich vorzugsweise in der Tür, sofern es sich um eine vollständig öffenbare Gehäusewand handelt Abbildung 2 (A) bis (F) zeigt verschiedene Ausgestaltungen der erfindungsgemäßen Schleuse. Typischerweise umfasst die Schleuse einen Rahmen, dehnbare, elastische Bänder, eine Dichtung vorzugsweise aus Moosgummi, und eine Abdeckung mit einem Magneten.

In einer bevorzugten Ausführungsform kann eine solche Schleuse (beispielsweise zur Probennahme) direkt durchgriffen werden, ohne dass es effektiv zu einem Gasaustausch zwischen Kammer und Umgebung kommt, und ohne, dass die Schleuse erst durch zusätzliche Handlungsschritte geöffnet werden müsste, wodurch der für die Erfassung der Transienten der Metabolite erforderliche Takt der Probennahme von 2-10 Sekunden ermöglicht wird. Besonders bevorzugt setzt sich eine solche Schleuse aus mehreren, sich kreuz- oder sternförmig überlappenden elastischen Bändern zusammen (s. Abbildung 2(B) und (D)), welche über die Öffnung in der Gehäusewand aufgespannt sind.

Als Materialien für die dehnbaren, elastischen Bänder eignen sich beispielsweise Nitril, Kautschuk oder weitere. In einer bevorzugten Ausführungsform bestehen die dehnbaren, elastischen Bänder aus Latex.

In einer besonders bevorzugten Ausführungform können zwei oder mehrere solcher Schleusen hintereinander verbaut werden, wobei ein Luftschleier/Schutzgasstrom zwischen den einzelnen Schleusen eine nahezu vollkommene Verhinderung des Gasaustausches gewährleisten kann.

In einer weiteren Ausführungsform umfasst die erfindungsgemäße Kammer zwei separate Schleusen, durch welche beispielsweise eine Person mit beiden Händen in der Kammer agieren kann oder auch zwei Personen simultan oder alternierend Proben nehmen können, wodurch beispielsweise Proben von unterschiedlichen pflanzlichen Strukturen (wie beispielsweise Blatt und Blüte) gleichzeitig genommen werden können sowie auch das Intervall zwischen zwei Probennahmen weiter verkürzt werden kann.

Vorzugsweise umfasst die Schleuse mindestens zwei, besonders bevorzugt mindestens vier, ganz besonders bevorzugt sechs oder acht, sich kreuz- oder sternförmig überlappenden dehnbaren Bänder, bevorzugt aus Latex (s. Abbildung 2(B) und (D)), welche idealerweise eine aufgerauhte Oberfläche, beziehungsweise einen möglichst geringen Reibungskoeffizienten, aufweisen, um optimale Gängigkeit der Hand sowie der Proben durch die Schleuse zu gewährleisten.

In einer besonders bevorzugten Ausführungsform sind die Latexbänder mittels eines Klemmmechanismusses (s. Abbildung 2(E) und 2(F)) auswechselbar auf einen zweiteiligen Rahmen, bestehend aus einer mit einer Durchgreiföffnung (18) versehenen Basisplatte (16) zur Befestigung der Latexbänder und einer zweiten, kleineren und ebenfalls mit einer Durchgreiföffnung versehenen Platte (sechs-oder achteckig; (17)) zur Auflage und Ausrichtung der Latexbänder aufgespannt.

Um die Kammer zwischen Experimenten, während des Gasaustausches oder bei Versuchsanordnungen, die keine wiederholte Probennahme in kurzen Zeitabständen erfordern, vollkommen (und druckdicht) zu verschliessen, ist eine Abdeckung der durch die Schleuse geschützten Öffnung vorteilhaft. Eine solche Abdeckung kann beispielsweise durch eine Platte geschehen, welche aus dem selben Material bestehen kann wie die Gehäusewände der Kammer, eine Haltevorrichtung aufweisen kann und über eine Befestigungsvorrichtung, beispielsweise Schrauben, Profile, eine Steckvorrichtung oder Magneten, an der Gehäusewand befestigt werden kann, so dass die Schleuse dicht verschlossen ist.

In einer bevorzugten Ausführungsform besteht die Schleusenabdeckung aus Polycarbonat, ist mit einem Haltegriff versehen und mit Magneten am Rahmen der Schleuse zu befestigen.

Die Gehäusewände bestehen vorzugsweise aus transparenten Materialien, die eine hohe Lichtdurchlässigkeit bei möglichst geringer Abwandlung der spektralen Zusammensetzung des eintretenden Lichtes aufweisen. Damit können künstliche Lichtquellen vermieden werden und die Beleuchtungsbedingungen innerhalb der Kammer verschiedenen experimentellen Bedingungen angepasst werden. Als geeignete Materialien kommen beispielsweise Glas und Kunststoffe wie Acrylglas oder Polyvinylchlorid und Polycarbonat in Frage. Bevorzugt bestehen die Gehäusewände aus Polycarbonat.

Die Wanddicke der einzelnen Gehäusewände kann unterschiedlich sein.

In einer bevorzugten Ausführungsform weisen die Gehäusewände eine Wanddicke von 3 bis 11, besonders bevorzugt von 5 bis 9 mm auf.

Zur einfachen Handhabung der Kammer ist die Verwendung leichter Materialien bevorzugt. Für den Gehäuserahmen eignen sich verschiedene Materialien wie beispielsweise Holz, Kunststoffe mit beigemischten Holzfasern, Kunststoffe, Metalle wie Edelstahl, besonders verschiedene Aluminiumlegierungen. In einer besonders bevorzugten Ausführungsform besteht der Gehäuserahmen aus Aluminium, bevorzugt Duraluminium.

Um zuverlässige Messergebnisse zu erhalten, ist es - wie bereits beschrieben - essentiell, dass zwischen der Kammer und der äußeren Umgebung möglichst geringer oder kein Luftaustausch stattfindet. Daher ist es zweckmäßig, wenn der Gehäuserahmen zusätzlich mit einem Abdichtungsmaterial versehen ist, welches ein luftdichtes Verschließen der Kammer ermöglicht. Geeignete Abdichtungsmaterialien sind beispielsweise (Neopren), Acryl oder Silicon, wobei Materialien mit einem geringen Lösungsmittelgehalt zu bevorzugen sind. In einer bevorzugten Ausführungsform besteht das Abdichtungsmaterial aus essigvernetzendem Silicon ohne (fungizide) Beimischungen (sog. Aquariensilikon).

Um ein luftdichtes Schließen der Tür sowie der Schleusenabdeckung zu ermöglichen, eignen sich verschiedene Lösungen wie beispielsweise Gummiprofile oder Schaumstoffpolster, Zellkautschuk. Bevorzugt werden Tür und Abdeckung mittels auf das Gehäuse aufgebrachten Rahmen aus geschlossenporigem Schaumstoff wie Neopren - Moosgummi abgedichtet.

Um die Reaktorkammer, also das obere Modul der bevorzugten Ausführungsform der Kammer, luftdicht auf die Luftregulationskammer, also das untere Modul der Kammer, aufzusetzen, eignen sich ebenfalls verschiedene Lösungen wie beispielsweise Gummiprofile oder Schaumstoffpolster, Zellkautschuk.

Bevorzugt wird die Reaktorkammer, also das obere Modul der bevorzugten Ausführungsform der Kammer, luftdicht mit der Luftregulierungskammer, also dem unteren Modul der Kammer, verbunden, indem zwischen beiden Modulen ein umlaufender Streifen aus Neopren-Moosgummi angebracht ist. In einer besonders bevorzugten Ausführungsform wird das obere Modul der Kammer mit vier Zentrierzapfen in Position gehalten, wobei ein umlaufender Streifen Neopren-Moosgummi für eine luftdichte Verbindung der beiden Kammermodule sorgt.

Das zu analysierende Pflanzenmaterial sollte während des Versuchs kontrollierten Umweltbedingungen wie Temperatur, Feuchtigkeit, Druck und Luftzufuhr ausgesetzt sein. Zu diesem Zweck befindet sich vorzugsweise unterhalb der Reaktorkammer (1) die Luftregulationskammer (2), welche eine Temperaturregelungseinheit mit Belüftungsvorrichtung (7) eine Luftbefeuchtungseinheit (8) und eine Gasabsorptionseinheit (9) umfasst.

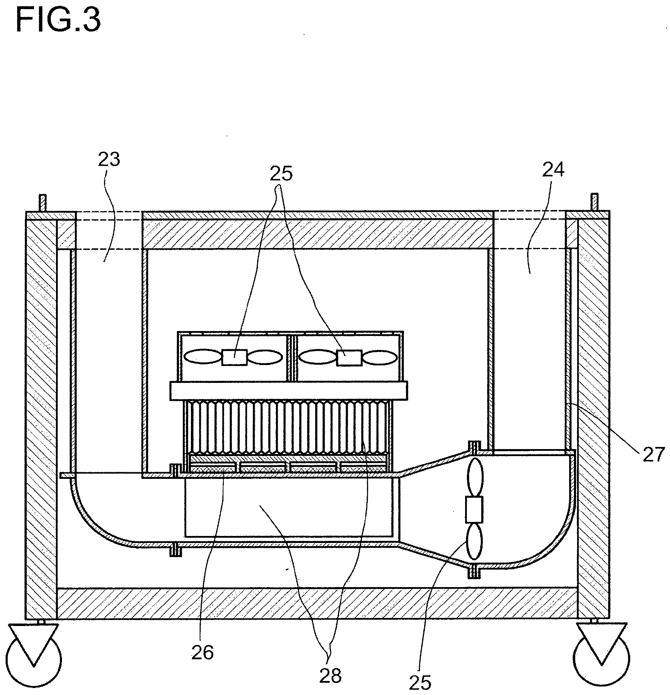

In der Temperaturregelungseinheit mit Belüftungsvorrichtung (Abb. 3) befindet sich mindestens ein Ventilator oder Lüfter, bevorzugt zwei Ventilatoren / Lüfter. Der Umluftstrom richtet sich nach dem Volumen der Markierungskammer und soll eine effiziente Durchmischung der Gase ermöglichen. Angestrebt ist hierbei eine eher schwache ("sanfte") Luftbewegung innerhalb der Kammer bei hohem Luftdurchsatz, was sich bevorzugt durch eine relative Vergrösserung der Öffnungen des Umluftkanals und den Einsatz mehrerer Ventilatoren erreichen lässt.

In einer bevorzugten Ausführungsform beträgt der Umluftstrom etwa 1000L Luft pro min.

Zur Temperaturregulation befindet sich im unteren Teil des Gehäuses ein Peltier-Element mit Kühlung, in einer bevorzugten Ausführungsform mit einem Aluminiumrippen-Kühlkörper mit einem oder mehreren Ventilatoren. In einer besonders bevorzugten Ausführungsform weist das Peltier-Element eine Leistungsaufnahme von 380 Watt auf.

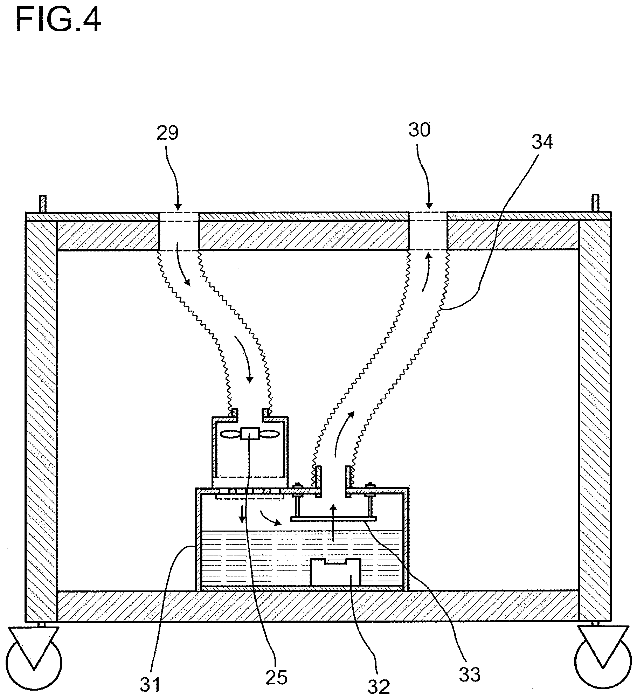

Die Luftbefeuchtungseinheit (Abb. 4) umfasst vorzugsweise ein mit Wasser gefülltes Wassergefäß, in welchem sich ein (piezokeramischer) Ultraschallwandler befindet. Um Ultraschallwellen für die Luftbefeuchtung nutzen zu können, muss elektrische Energie in mechanische Energie umgewandelt werden. Das erfolgt in dem piezoelektrischen Wandler (auch Transducer und Schwinger genannt). Piezokeramische Ultraschallwandler sind aus dem Stand der Technik bekannt (siehe z.B: AIRWIN. Für eine effektive Wasservernebelung und somit Befeuchtung der zugeführten Luft ist eine Frequenz des Ultraschallwandlers von beispielsweise 2 MHz vorteilhaft. Wie in Abbildung 4 dargestellt, ist die Luftbefeuchtungseinheit über Schläuche für Lufteintritt bzw. -austritt mit der Reaktorkammer verbunden.

Wie zuvor geschildert, ist es für eine fehlerfreie Versuchsanordnung erforderlich, dass in der Kammeratmosphäre vorhandenes nicht-markiertes Kohlendioxid möglichst vollständig aus der Kammer entfernt und gebunden wird, so dass das zu analysierende Pflanzenmaterial überwiegend markiertes ¹³CO₂ aufnehmen und messbar in den Stoffwechsel einbauen kann.

Folglich enthält die Gasabsorptionseinheit (siehe Abb. 5 A) vorzugsweise ein Material, welches Kohlendioxid absorbieren kann, besonders bevorzugt ein Gemisch aus NaOH / KOH und CaOH (sog. Atemkalk). Verschiedene Rezepturen solcher Kohlendioxidabsorber sind aus dem Stand der Technik bekannt. In der Regel ist dem Absorbermaterial (38) ein Indikator zugesetzt, welcher die CO₂-Sättigung / den Verbrauchszustand des Absorbermaterials durch einen Farbumschlag visualisiert.

In einer besonders bevorzugten Ausführungsform ist das absorbierende Material Atemkalk. In einer bevorzugten Ausführungsform kommen etwa 15L Absorbermaterial (38) zum Einsatz.

Bevorzugt wird die bereinigte Luft über einen Feinstaubfilter (39) geleitet, nachdem sie das Absorbermaterial passiert hat.

In einer bevorzugten Ausführungsform hat die Pumpe, welche die Luft über die Ansaugöffnung aus der Kammer ansaugt und über das Absorbermaterial leitet, eine Leistung, welche zwischen 1000 und 3000, besonders bevorzugt schaltbar zwischen 1800 und 2500 Liter Luft pro Minute durchsetzen kann. In einer besonders bevorzugten Ausführungsform ist die Pumpe eine Kreiselpumpe mit schaltbar entweder 1800 oder 2500 l/min Luftdurchsatz.

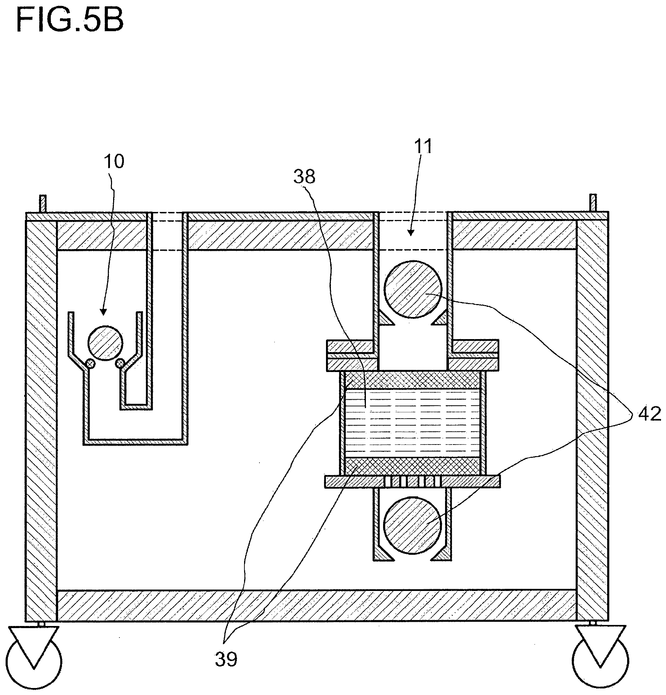

Um einen Unterdruck während des Austauschvorganges zu vermeiden (siehe Abb 5 (B)), befindet sich im Boden der Markierungskammer eine Einströmöffnung (= Unterdruckventil, siehe Abb 5 (B) rechte Seite), bzw. Nr. (11) in Abb. 1), welche ebenfalls mit einem Feinstaubfilter (39) und einem CO₂-Absorber (38) versehen ist, so dass die durch den Unterdruck aus der Umgebung der Reaktorkammer einströmende Luft von CO₂ bereinigt ist.

Der Ausgleich eines nach dem Abschalten der Pumpe zur Gasarbsorbtionseinheit kurzzeitig auftretenden Überdrucks wird durch ein ebenfalls im Boden der Reaktorkammer befindliches Kugelventil (= Überdruckventil; Abb. 5 (B) linke Seite, bzw. Nr. (10) in Abb.1) ausgeglichen, durch welches Luft aus der Reaktorkammer ausströmen, aber keine Luft in die Reaktorkammer eindringen kann. Diese Kombination von Einströmöffnung/Unterdruckventil mit CO₂-Absorber und Auslass-Kugelventil / Überdruckventil ermöglicht eine Durchführung der Markierungsexperimente unter Druckverhältnissen, welche weitestgehend denjenigen der Umgebung entsprechen ("Normaldruck").

Um einen möglichst gleichmäßigen Gehalt an ¹³CO₂ kontrollieren zu können, weist die erfindungsgemäße Markierungskammer ferner noch eine Vorrichtung zur Messung von Kohlendioxid auf. Hierzu eignen sich verschiedene, aus dem Stand der Technik bekannte Quellempfänger, beispielsweise Infrarot-Sensoren, photoakustische Sensoren oder Massenspektrometer. Vorzugsweise können die Konzentrationen an ¹²C und ¹³C getrennt bestimmt / voneinander abgegrenzt werden.

In einer bevorzugten Ausführungsform handelt es sich bei der CO₂-Messvorrichtung um ein Massenspektrometer, mit welchem sich separate Messwerte für ¹²CO₂ und ¹³CO₂ bestimmen lassen.

In einer besonders bevorzugten Ausführungsform handelt es sich bei der CO₂-Messvorrichtung um ein Massenspektrometer zur Analyse eines Massenbereichs von 1 bis 100 amu (atomic mass units).

### (2) Markierung von Pflanzen

Die Verteilungen metabolischer Flüsse geben Rückschlüsse auf Stoffwechselreaktion und Engpässe im Pflanzenstoffwechsel und werden als Ausgangspunkte für ,rational metabolic Engineering' sowie zur vertiefenden Analyse des Pflanzenstoffwechsels verwendet. Mit dem erfindungsgemäßen Verfahren kann der Stoffwechsel einer Pflanze auch unter Stressbedingungen analysiert werden, zB nach der Behandlung mit einem Pflanzenschutzmittel, nach der Behandlung mit Kälte, Trockenheit, Salz, etc. Weiterhin kann auch der Einfluss einer genetischen Veränderung auf den Pflanzenstoffwechsel analysiert werden.

Die Markierungsexperimente werden bevorzugt unter physiologischen bzw. natürlichen CO₂-Konzentrationen durchgeführt. Damit verbunden ist eine erforderliche ¹³CO₂-Konzentration von etwa 360 bis 420, bevorzugt etwa 380 bis 400 ppm im Reaktor. Hierzu muss das natürliche CO₂ in der Kammer vor dem Experiment möglichst vollständig entfernt werden. Dazu werden zunächst die Pflanzen in der Kammer platziert und die Türe geschlossen, sodass keine Luft mit der Umgebung ausgetauscht werden kann. Bevorzugt werden Nutzpflanzen wie bespielsweise Mais, Soja, Reis, Raps, Baumwolle, Weizen verwendet, besonders bevorzugt Mais oder Reis. Die zu markierenden Pflanzen können sich in verschiedenen Entwicklungsstadien wie Embryonalstadium, vegetatives Stadium oder generatives Stadium, befinden.

Bevorzugt befinden sich die Pflanzen in einem der folgenden Stadien: Keimung/Austrieb, Blattentwicklung, Bestockung, Schossen, Ähren- bzw. Rispenschwellen, Ähren- bzw. Rispenschieben, Blüte, Fruchtentwicklung, Frucht- bzw. Samenreife, Absterben oder Eintreten der Vegetationsruhe. Besonders bevorzugt befinden sich die zu markierenden Pflanzen in einem der folgenden Stadien: Blattentwicklung, Bestockung, Schossen, Ähren- bzw. Rispenschwellen, Ähren- bzw. Rispenschieben, Blüte, Fruchtentwicklung, Frucht- bzw. Samenreife.

In einer bevorzugten Ausführungsform werden mehrere Pflanzen, welche sich im selben Entwicklungsstadium befinden, simultan markiert.

In einer weiteren bevorzugten Ausführungsform werden mehrere Pflanzen, welche sich in unterschiedlichen Entwicklungsstadien befinden, simultan markiert.

In einer weiteren bevorzugten Ausführungsform handelt es sich bei den mit dem erfindungsgemäßen Verfahren zu markierenden Pflanzen um transgene Pflanzen.

In einer bevorzugten Ausführungsform werden mindestens 16 Reissetzlinge im Alter von 12 Tagen nach Keimung simultan markiert, indem sie mit ihren Kulturgefässen in der Reaktorkammer untergebracht werden.

In einer weiteren Ausführungsform werden die Pflanzen zusätzlich zur ¹³C-Markierung mit ¹⁵N markiert, indem sie innerhalb der Reaktorkammer in einem Gefäß für hydroponische Kultur, welches ein mit ¹⁵N markiertes Nährstoffsalz enthält, platziert werden.

In einer bevorzugten Ausführungsform werden die Pflanzen simultan mit ¹³C und ¹⁵N markiert, wobei die Applikation beider Markierungspulse zeitgleich erfolgt.

Das Entfernen des CO₂ erfolgt vor dem Experiment über einen CO₂-Absorber, bevorzugt mit Atemkalk. Der CO₂-Absorber ist außerhalb platziert und über die Kunststoffleitungen mit dem Gehäuse verknüpft. Die Luft aus dem oberen Teil der Kammer wird mittels einer Pumpe angesaugt und gleichzeitig, nach erfolgter Absorption, wird die CO₂-freie Luft der unteren Kammer, direkt ausgerichtet zum Ventilator, zugeführt. Der Austausch erfolgt in weniger als 60 Sekunden, bevorzugt in weniger als 45 Sekunden, besonders bevorzugt in weniger als 30 Sekunden. Direkt im Anschluss erfolgt die Injektion der entsprechenden Menge des ¹³CO₂ Gases über eine Spritze. Durch Positionierung des Injektionsventils direkt über der Auslassöffnung des Umluftkanals erfolgt eine optimale Durchmischung im Sinne einer gleichmäßigen Verteilung des ¹³CO₂ auf das Volumen der Reaktorkammer und somit eine Einstellung der gewünschten ¹³CO₂-Konzentration.

Entsprechend des experimentellen Aufbaus wird möglichst zeitgleich mit der Umstellung der Kammeratmosphäre auf ¹³CO₂ oder kurz darauf mit der Probennahme begonnen, woraufhin eine regelmäßige Probennahme erfolgt. Bevorzugt erfolgt eine Probennahme alle 2 bis 20 Sekunden, bevorzugt alle 2 bis 10 Sekunden, besonders bevorzugt alle 2 bis 5 Sekunden für mindestens die ersten 60 Sekunden. Bevorzugt erfolgen weitere Probennahmen in Intervallen von beispielsweise 2, 5, 10, 20 , 30, 40, 50, 60, 70, 80, 90, 120, 180, 240, 300 Sekunden, wobei mindestens ein Zeitraum von 900 Sekunden ab der ersten Probennahme durch regelmäßige Probennahmen erfasst wird.

In einer bevorzugten Ausführungsform wird die erste Probe zeitgleich mit der Applikation des ¹³CO2 genommen. Weitere Proben werden jeweils 10, 20, 30, 40, 50, 60, 90, 120, 150, 180, 240, 300, 420, 600, sowie 900 Sekunden nach Applikation des ¹³CO₂ genommen.

In einer weiteren Ausführungsform kann die Probennahme jedoch auch über einen deutlich längeren Zeitraum, beispielsweise von mehreren Stunden, Tagen oder Wochen erfolgen.

Bevorzugt wird das erfindungsgemäße Verfahren mit identischen Probennahmeintervallen für jedes Experiment mehr als einmal, besonders bevorzugt zweimal, dreimal oder häufiger, wiederholt.

Das restliche in der Kammer verbleibende ¹³CO₂ kann vor Beginn des nächsten Experiments über den CO₂- Absorber entfernt werden.

Die vorliegende Erfindung wird anhand der folgenden Beispiele erläutert ohne darauf beschränkt zu sein

### BEISPIELE

### BEISPIEL 1: Generelles Verfahren

Eine Pflanze wird in einer erfindungsgemäßen Isotopenmarkierungskammer mit einem oder mehreren stabilen Isotopenlabeln markiert. Die Verteilung des Isotopenlabels in der Pflanze wird zu verschieden Zeitpunkten nach dem Markierungspuls im gefriergetrockneten und gemahlenen Pflanzenmaterial analysiert. Die Probennahme nach dem Markierungspuls erfolgt aus der erfindungsgemäßen Isotopenmarkierungskammer mittels einer Schleuse, wodurch eine Veränderung der Atmosphäre in der erfindungsgemäßen Isotopenmarkierungskammer während der Probennahme verhindert wird. Die Labelverteilung in einer Probe wird mittels Massenspektrometrie nach chemischer Extraktion der Pflanzenmaterialien analysiert. Aus der Labelverteilung können Flüsse im Stoffwechsel von ganzen Pflanzen mittels mathematischer Modellierung berechnet werden.

### BEISPIEL 2: Material und Methoden

### 2.1 Zubehör für die Durchführung des Markierungsexperiments

- Erfindungsgemäße Isotopenmarkierungskammer
- Gasdichte Beutel zum Transport und der Lagerung von ¹³CO₂
- Gasdichte Spritze
- ¹³CO₂ in Gasflasche mit Druckminderer
- ¹⁵NH₄NO₃ (optional)
- Hydroponisches System (Wanne mit Styropor-Lochplatte)
- Pflanzgefässe
- Erde
- Pflanzgranulat
- Pflanzen

### 2.2 Zubehör für Probennahme und Zerkleinerung

- Waage
- Flüssiger Stickstoff und geeignete Wanne
- Erntegefässe (2ml Eppendorff Vials, Falcon Tubes unterschiedlicher Größe)
- Festhalte-Schere
- Festhalte-Zange
- Pinzette
- Schwingmühle
- Mahlbecher mit Kugeln (Durchmesser 2-15 mm)
- Tiefkühlschrank -80°C

### 2.3 Zubehör für Probenextraktion

- Fast Prep mit Trockeneiseinsatz
- Trockeneis
- Zentrifugenfilter
- Zentrifuge
- Gefriertrocknung
- Messgefäße
- Eppendorff-Gefäße
- Falcon-Gefäße

### 2.4 Zubehör für Probemessung

- UPLC
- Massenspektrometer

### 2.5 Chemikalien

- Lösemittel (Ethanol, Acetonitril, Wasser, Dichlormethan)
- Ammoniumacetat
- Tributhylammonium
- Essigsäure
- Interne Standards
- Testsubstanzen

### BEISPIEL 3: Analytische Vorgehensweise

### 3.1 Markierungsexperiment

Das Markierungsexperiment wird durchgeführt zur Bestimmung metabolischer Flüsse in ganzen Pflanzen. Die Verteilungen metabolischer Flüsse geben Rückschlüsse auf Stoffwechselreaktion und Engpässe im Pflanzenstoffwechsel und werden als Ausgangspunkte für ,rational metabolic Engineering' sowie zur vertiefenden Analyse des Pflanzenstoffwechsels verwendet. Mit dem erfindungsgemäßen Verfahren kann der Stoffwechsel einer Pflanze auch unter Stressbedingungen analysiert werden, zB nach der Behandlung mit einem Pflanzenschutzmittel, nach der Behandlung mit Kälte, Trockenheit, Salz, etc. Weiterhin kann auch der Einfluss einer genetischen Veränderung auf den Pflanzenstoffwechsel analysiert werden. Zur Untersuchung der Flüsse in einer Pflanze wird diese mit einem stabilen Isotop gelabelt. Das Isotopenlabel wird im Pflanzenstoffwechsel verfolgt. Basierend auf der Labelverteilung können Rückschlüsse auf den Metabolismus der untersuchten Pflanze gezogen werden. Soll eine Stressbedingung analysiert werden, kann diese zu einem beliebigen Zeitpunkt vor oder während des Markierungsexperiments appliziert werden.

### 3.2 ¹³CO₂ Markierung einer ganzen Pflanze

Pflanzen in unterschiedlichen Entwicklungsstadien werden in der erfindungsgemäßen Isotopenmarkierungskammer platziert. Die lichtdurchlässige Isotopenmarkierungskammer befindet sich in einer für das Pflanzenwachstum geeigneten Bereich, zB. einer Phytokammer, einem Gewächshaus, unter freiem Himmel, etc. Dadurch ist eine ausreichende Lichtzufuhr durch die transparente Kammerwand gewährleistet. Die erfindungsgemäße Isotopenmarkierungskammer ist luftdicht zur äußeren Atmosphäre abgedichtet. Luftfeuchtigkeit und Temperatur werden entsprechend den Anforderungen der untersuchten Pflanzenart (bzw. des Experiments) eingestellt. Vor der Durchführung des Markierungsexperiments wird ambientes ¹²CO₂ mit Hilfe eines Absorbers aus der in der Kammer befindlichen Luft entfernt. Anschließend wird die gewünschte ¹³CO₂ Konzentration (zB 400 ppm) in der Kammer eingestellt. Der in der Kammer befindliche Luftstrom stellt eine gleichmäßige Verteilung des ¹³CO₂ in der Kammer innerhalb weniger Sekunden sicher. Die ¹³CO₂ Inkubationsdauer wird als Markierungspuls bezeichnet. Typischerweise werden Markierungspulse zwischen 2 Sekunden und 180 Minuten durchgeführt, längere Pulsexperimente sind ebenfalls möglich. Nach Einstellung der Atmosphäre auf ¹³CO₂ werden Proben aus der Kammer genommen, bzw. ganze Pflanzen aus der Kammer geerntet (bei nur minimaler Veränderung der Atmosphäre in der Kammer). Darüber hinaus besteht die Möglichkeit, Pflanzen nach dem Markierungsexperiment zur weiteren Entwicklung in regulärer Umgebung und Atmosphäre (zB Phytokammer, Gewächshaus, etc.) aufzubewahren und erst zu einem späteren Zeitpunkt zu ernten

### 3.3 ¹⁵N-Markierung ganzer Pflanzen

Zur Applikation eines zusätzlichen ¹⁵N-Markierungspulses werden Pflanzen in einem hydroponischen System angezogen. Das hydroponische System enthält eine Nährlösung mit Stickstoffquelle (zB NH₄NO₃). Zum Zeitpunkt des Experiments werden die Pflanzen auf ein zweites hydroponisches System überführt, in dem die verfügbare Stickstoffquelle (zB NH₄NO₃) durch ¹⁵N-haltige Verbindungen ersetzt wurde (zB ¹⁵NH₄¹⁵NO₃, ¹⁴NH₄¹⁵NO₃, ¹⁵NH₄¹⁴NO₃). Nach Ende des Markierungspulses werden die Pflanzen aus dem hydroponischen System entnommen, mit regulärer Nährlösung abgespült und zur weiteren Entwicklung in ein reguläres hydroponisches System überführt, das Stickstoffverbindungen in Form von ¹⁴N-Stickstoff enthält. Alternativ werden Pflanzen direkt nach dem Markierungspuls geerntet. Die Applikation eines ¹⁵N-Markierungspulses kann in Kombination mit der ¹³CO₂ Markierung der ganzen Pflanze erfolgen

### BEISPIEL 4: Pflanzenernte

Pflanzen werden während des Markierungsexperiments aus der Kammer geerntet. Zu diesem Zweck wurde eine variable Schleuse in die Kammer eingebaut. In der Kammerwand befindet sich eine Öffnung, auf die der Rahmen der erfindungsgemäßen Schleuse (mit Moosgummidichtungen) dicht aufgesetzt und verschraubt wird. Die Schleuse besteht aus einem Rahmen mit Gummibändern, die einen minimalen Eingriff in die Kammer von außen ermöglichen (Abb. 2 (A)-(F)). Soll die Schleuse über einen längeren Zeitraum nicht genutzt werden, bzw. besteht die Notwendigkeit, die Kammer druckdicht zu verschließen (zB zur Entfernung von ambientem ¹²CO₂), kann die Schleuse mit einer transparenten Abdeckung verschlossen werden. Diese Abdeckung wird mit Magnetverschlüssen dicht auf die Schleuse aufgesetzt. Pflanzenmaterial wird entsprechend des experimentellen Designs geerntet und sofort in flüssigem Stickstoff tiefgekühlt. Der Stickstoffbehälter befindet sich dabei außerhalb der Kammer. Die Ernte kann bereits wenige Sekunden nach Beginn des Markierungsexperiments beginnen. Zur Ernte wird eine sogenannte Rosenpräsentierschere eingesetzt. Dabei handelt es sich um eine Schere oder Zange, die die Pflanze, bzw. Pflanzenteile abschneidet und gleichzeitig greift (bzw. festhält). Bei der Ernte können ganze Pflanzen oder verschiedene Pflanzenteile separat geerntet und eingefroren werden (zB Fahnenblatt, Blatt, Stängel, Ähre, Spikelet, Samen, Wurzel, Seedling). Alle Pflanzen/Pflanzenteile werden nach der Ernte bei -80°C aufbewahrt.

### BEISPIEL 5: Probenvorbereitung

### 5.1 Probenzerkleinerung

Tiefgekühltes Pflanzenmaterial wird mittels einer Schwingmühle zerkleinert. Dafür werden die für das Pflanzenmaterial geeigneten Mahlbecher und Mahlkugeln (Durchmesser 2-15 mm) verwendet.

Beispiel:
Seedling: 2 ml Eppendorf Vial, 2 Stahlkugeln,
Wurzel: 2 ml Eppendorf Vial, 2 Stahlkugeln,
Blatt: 20 ml Szintillationsgefäss im Edelstahlmahlbecher, 5 Stahlkugeln,
Ähren: Edelstahlmahlbecher, 1 Stahlkugel,

Zerkleinertes Pflanzenmaterial wird gefriergetrocknet bei Raumtemperatur unter Argon aufbewahrt.

### 5.2 Probenextraktion (NRJ Methode wie in WO2011/003945 offenbart) mit geringen Anpassungen)

10 mg getrocknetes Pflanzenmaterial wird eingewogen und mit 900 uL einer -80 °C kalten Dichlormethan/Ethanol (2:1 v/v)-Lösung versetzt. Anschließend wird die Suspension mit 150 µL, 1.5 M AmOAc überschichtet und die Probe unter Verwendung einer FastPrep (gekühlt mit Trockeneis) mit Glaskugeln extrahiert. Nach der ersten Extraktion (30 s FastPrep, 6.5 m/s) wird die Probe 2 min bei 14000 rpm und 0 °C zentrifugiert. Im Anschluss werden 100 µl der oberen Phase abgenommen und in Zentrifugenfilter überführt. Anschließend werden 150 µL 1.5 M AmOAc-Lsg zugesetzt und es wird ein zweites Mal extrahiert (30 s FastPrep (Cryo mit Trockeneis), 6.5 m/s). Nach erneuter Zentrifugation bei 14000 rpm und 0 °C werden 200 µl der oberen Phase mit dem ersten Extrakt im Zentrifugenfilter vereinigt und 5 min bei 14000 rpm und 0 °C zentrifugiert. 240 µL Filtrat werden in Messgefäße überführt und zwei Tage gefriergetrocknet. Nach vollständiger Gefriertrocknung werden die Proben in 100 µl des Laufmittels A rekonstituiert und mittels der NRJ Methode (Ionenpaarchromatographie, UPLC-LC-MS/MS) analysiert.

### BEISPIEL 6: Auswertung der Isotopenverhältnisse

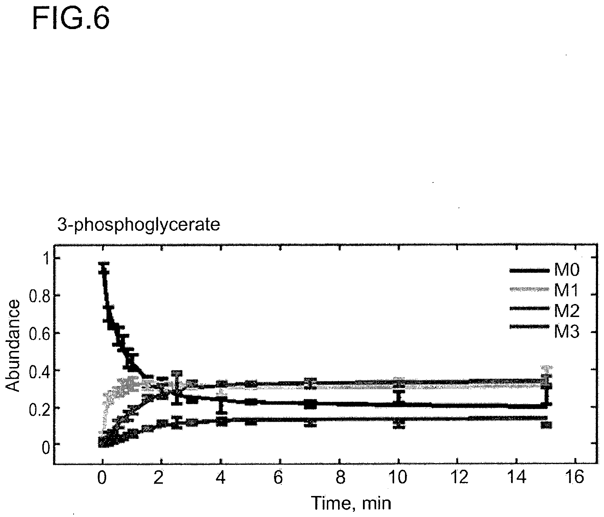

Bei der LC-MS/MS Messung (wobei LC-MS/MS für Liquid-Chromatographie-Massenspektometrie/Massenspektometrie steht) werden für alle Analyten MRM Übergänge (wobei MRM "multiple reaction monitoring" bedeutet für alle Isotopomere aufgezeichnet. Die Flächen oder Höhen der jeweiligen, die einzelnen Isotopomere repräsentierenden Peaks werden durch Integration bestimmt. (Abb. 6)

Die Peakflächen der Isotopomere werden als Funktion des Probennahmezeitpunkts aufgetragen. Dabei werden in der Regel eine Abnahme bzw. eine Zunahme von Isotopomeren beobachtet (Transienten) (Abb. 6).

Durch einen Vergleich theoretisch berechneter und experimentell aufgezeichneter Transienten können reale Flüsse mithilfe eines stöchiometrischen Modells des Pflanzenstoffwechsels berechnet werden.

### BEISPIEL 7: Flussanalysen mit ganzen Reis-Pflanzen

Ganze Reispflanzen (Seedlings - Setzlinge) wurden in einer erfindungsgemäßen Markierungskammer für mit ¹³CO₂ markiert. Hierzu wurden 16 Reissetzlinge als Ganzes in ihren Pflanzgefässen in einer Gewächshauskammer für einen Zeitraum von 10 Tagen bei normaler Atmosphäre akklimatisiert.

Zu Beginn des Experimentes wurden die Pflanzen in der Markierungskammer plaziert. Anschliessend wurde die in der Kammer befindliche Atmosphäre von ambientem ¹²CO₂ bereinigt, indem sie für einen Zeitraum von 45 Sekunden bei einem Luftstrom von 2500l/min durch die Gasabsorptionseinheit geleitet wurde. Hierdurch wurde der CO₂-Gehalt der bereinigten Luft auf etwa 20ppm gesenkt. Anschließend wurde der ¹³CO₂-Gehalt der Kammeratmosphäre auf 400ppm eingestellt, indem sofort 300 ml ¹³CO₂ durch den Injektionskanal in die Kammer gegeben wurden.

Um die oberirdische grüne Biomasse der einzelnen Reissetzlinge möglichst vollständig als Probe zu gewinnen, wurden die Reissetzlinge als Ganzes (ohne Wurzeln) möglichst knapp über der Erdoberfläche des Pflanzgefässes mit einer Präsentierschere abgeschnitten, durch die Schleuse entnommen und sofort in flüssigem Stickstoff tiefgekühlt. Dieser Prozess ließ sich durch die Verwendung der erfindungsgemässen Kammer innerhalb von 1 bis 2 Sekunden durchführen.

Die erste Probe wurde zeitgleich mit der Applikation des ¹³CO₂ genommen. Weitere Proben wurden jeweils 10, 20, 30, 40, 50, 60, 90, 120, 150, 180, 240, 300, 420, 600, sowie 900 Sekunden nach Applikation des ¹³CO₂ genommen.

Die resultierenden 16 Proben wurden wie in Beispiel 5 beschrieben aufgearbeitet. Das Experiment wurde insgesamt dreifach wiederholt, so dass für jeden der 16 Zeitpunkte drei Proben für die Messung der ¹³CO₂-Inkorporation in die verschiedenen Metaboliten zur Verfügung standen. Wie in Beispiel 6 beschrieben, konnten aus den gemittelten Messwerten der jeweiligen verschiedenen Isotopomere Transienten für folgende Metaboliten berechnet werden: 3-Phosphoglycerat (s. Abb. 6), Citrat, Malat, Ribose-5-Phosphat, Ribulose-5-Phosphat, Fructose-6-Phosphat, Fructose-1,6-Bisphosphat, Isocitrat, Sedoheptulose-7-Phosphat, Glucose-6-Phosphat, Succinat, Dihydroxyacetonphosphat, Phosphoenolpyruvat.

Die exemplarischen Transientenkurven der verschiedenen Massenisotopomere von 3-Phosphoglycerat (Abb. 6:, M0, M1, M2, M3) verdeutlichen die Unabdingbarkeit der schnellen und häufigen Probennahme innerhalb der ersten 120 Sekunden nach der Umstellung der Atmosphäre auf ¹³CO₂, welche erst durch die erfindungsgemäße Markierungskammer und insbesondere die zur Probennahme verwendete Schleuse ermöglicht wurde:
Erst die Verwendung der erfindungsgemäßen Kammer mit Schleuse ermöglicht eine zeitliche Auflösung der Transientenverläufe - insbesondere von Metaboliten mit schneller Inkorporation von ¹³CO₂ (hohem Stoffumsatz) und somit der Inkorporation des markierten Kohlendioxides

Durch Abgleich der erhaltenen Daten mit einem isotopisch dynamischen Modell des pflanzlichen Stoffwechsels konnte erstmalig eine sogenannte Flux-Map für eine ganze Erntepflanze (einen Reissetzling) erstellt werden

## Patentansprüche

1. Isotopenmarkierungskammer zum Markieren von Stoffwechselprodukten in einem Organismus, umfassend eine Reaktorkammer (1), wobei die Reaktorkammer (1) die folgende Komponenten umfasst:
optional einen Gehäuserahmen (3), Gehäusewände (4), mindestens ein Injektionsventil (5), wobei mindestens eine Gehäusewand (4) vollständig und/oder teilweise öffenbar ist, **dadurch gekennzeichnet, dass** mindestens eine Gehäusewand eine Schleuse (6) aufweist und dass die Isotopenmarkierungskammer weiter eine Luftregulationskammer (2) umfasst, wobei die Luftregulationskammer **(2)** die folgende Komponenten umfasst:
eine Temperaturregelungseinheit **(7),** eine Luftbefeuchtungseinheit **(8),** und eine Gasabsorptionseinheit **(9).**

2. Isotopenmarkierungskammer nach Anspruch 1, **dadurch gekennzeichnet, dass** die Luftregulationskammer **(2)** außerdem ein Überdruckventil **(10)** und ein Unterdruckventil **(11)** aufweist.

3. Isotopenmarkierungskammer nach Anspruch 1 oder 2, **dadurch gekennzeichnet, dass** die mindestens eine vollständig und/oder teilweise öffenbare Gehäusewand eine Tür ist.

4. Isotopenmarkierungskammer nach einem der Ansprüche 1 bis 3, , **dadurch gekennzeichnet, dass** die Schleuse **(6)** Bestandteil der Tür oder einer der Wände ist.

5. Isotopenmarkierungskammer nach einem der Ansprüche 1 bis 4, **dadurch gekennzeichnet, dass** die Gehäusewände aus Polycarbonat bestehen.

6. Isotopenmarkierungskammer nach Anspruch 5, **dadurch gekennzeichnet, dass** die Gehäusewände eine Wanddicke von 3 bis 11, vorzugsweise 9 mm aufweisen.

7. Isotopenmarkierungskammer nach einem der Ansprüche 1 bis 6, **dadurch gekennzeichnet, dass** der Gehäuserahmen aus Aluminium besteht.

8. Isotopenmarkierungskammer nach einem der Ansprüche 1 bis 7, **dadurch gekennzeichnet, dass** der Gehäuserahmen zusätzlich mit einem Abdichtungsmaterial versehen ist.

9. Isotopenmarkierungskammer nach einem der Ansprüche 1 bis 8, **dadurch gekennzeichnet, dass** die Temperaturregulierungseinheit **(7)** mit mindestens einem Ventilator **(25)** versehen ist.

10. Isotopenmarkierungskammer nach einem der Ansprüche 1 bis 9, **dadurch gekennzeichnet, dass** die Luftbefeuchtungseinheit **(8),** einen piezokeramischen Ultraschallwandler **(32)** umfasst.

11. Isotopenmarkierungskammer nach einem der Ansprüche 1 bis 10, **dadurch gekennzeichnet, dass** die Gasabsorptionseinheit (9) Material enthält, welches Kohlendioxid absorbiert.

12. Isotopenmarkierungskammer nach Anspruch 11, **dadurch gekennzeichnet, dass** das Kohlendioxid-absorbierende Material Atemkalk ist.

13. Isotopenmarkierungskammer nach einem der Ansprüche 1 bis 12, **dadurch gekennzeichnet, dass** die Schleuse **(6)** mindestens zwei, sich kreuz- oder sternförmig überlappende, dehnbare Bänder **(20)** umfasst.

14. Verfahren zum Markieren von Pflanzen mit stabilen Isotopen, **gekennzeichnet durch** die Verwendung der Isotopenmarkierungskammer nach einem der Ansprüche 1 bis 13.

15. Verfahren nach Anspruch 14, **dadurch gekennzeichnet, dass** es sich bei den Pflanzen um Mais, Reis, Weizen, Soja oder Raps handelt.

## Claims

1. An isotope labeling chamber for labeling metabolites in an organism, comprising a reactor chamber **(1),** where the reactor chamber **(1)** comprises the following components:
optionally, a housing frame **(3),** housing walls **(4),** at least one injection valve **(5),** where at least one housing wall **(4)** can be opened fully and/or in part, wherein at least one housing wall has a lock (6), and wherein the isotope labeling chamber furthermore comprises an air regulation chamber (2), where the air regulation chamber **(2)** comprises the following components:
a temperature-regulating unit **(7),** an air humidification unit **(8)** and a gas absorption unit **(9).**

2. The isotope labeling chamber according to claim 1, wherein the air regulation chamber **(2)** additionally includes a pressure-relief valve **(10)** and a vacuum valve **(11).**

3. The isotope labeling chamber according to claim 1 or 2, wherein the at least one housing wall which can be opened fully and/or in part is a door.

4. The isotope labeling chamber according to any of claims 1 to 3, wherein the lock **(6)** is a component of the door or of one of the walls.

5. The isotope labeling chamber according to any of claims 1 to 4, wherein the housing walls are composed of polycarbonate.

6. The isotope labeling chamber according to claim 5, wherein the housing walls have a thickness of from 3 to 11, preferably 9, mm.

7. The isotope labeling chamber according to any of claims 1 to 6, wherein the housing frame is composed of aluminum.

8. The isotope labeling chamber according to any of claims 1 to 7, wherein the housing frame is additionally provided with a sealant.

9. The isotope labeling chamber according to any of claims 1 to 8, wherein the temperature-regulating unit **(7)** is provided with at least one blower **(25).**

10. The isotope labeling chamber according to any of claims 1 to 9, wherein the air humidification unit **(8)** comprises a piezoceramic ultrasonic transducer **(32).**

11. The isotope labeling chamber according to any of claims 1 to 10, wherein the gas absorption unit (9) comprises material which absorbs carbon dioxide.

12. The isotope labeling chamber according to claim 11, wherein the carbon-dioxide-absorbing material is soda lime.

13. The isotope labeling chamber according to any of claims 1 to 12, wherein the lock **(6)** comprises at least two expandable bands **(20)** which overlap in the shape of a cross or star.

14. A method for labeling plants with stable isotopes, in which the isotope labeling chamber according to any of claims 1 to 13 is employed.

15. The method according to claim 14, wherein the plants are maize, rice, wheat, soybeans or oilseed rape.

## Revendications

1. Chambre de marquage par des isotopes pour le marquage de produits métaboliques dans un organisme, comprenant une chambre de réacteur (1), la chambre de réacteur (1) comprenant les éléments suivants :
éventuellement un cadre (3) de boîtier, des parois (4) de boîtier, au moins une soupape d'injection (5), au moins une paroi (4) de boîtier pouvant être complètement et/ou partiellement ouverte, **caractérisée en ce qu'**au moins une paroi de boîtier présente un sas (6) et **en ce que** la chambre de marquage par des isotopes comprend en outre une chambre (2) de régulation de l'air, la chambre (2) de régulation de l'air comprenant les éléments suivants : une unité (7) de régulation de la température, une unité (8) d'humidification de l'air et une unité (9) d'absorption de gaz.

2. Chambre de marquage par des isotopes selon la revendication 1, **caractérisée en ce que** la chambre (2) de régulation de l'air présente en outre une soupape (10) de surpression et une soupape (11) de dépression.

3. Chambre de marquage par des isotopes selon la revendication 1 ou 2, **caractérisée en ce que** ladite au moins une paroi de boîtier pouvant être complètement et/ou partiellement ouverte est une porte.

4. Chambre de marquage par des isotopes selon l'une quelconque des revendications 1 à 3, **caractérisée en ce que** le sas (6) fait partie de la porte ou d'une des parois.

5. Chambre de marquage par des isotopes selon l'une quelconque des revendications 1 à 4, **caractérisée en ce que** les parois du boîtier sont constituées par du polycarbonate.

6. Chambre de marquage par des isotopes selon la revendication 5, **caractérisée en ce que** les parois du boîtier présentent une épaisseur de paroi de 3 à 11, de préférence de 9 mm.

7. Chambre de marquage par des isotopes selon l'une quelconque des revendications 1 à 6, **caractérisée en ce que** le cadre de boîtier est en aluminium.

8. Chambre de marquage par des isotopes selon l'une quelconque des revendications 1 à 7, **caractérisée en ce que** le cadre de boîtier est en outre pourvu d'un matériau d'étanchéité.

9. Chambre de marquage par des isotopes selon l'une quelconque des revendications 1 à 8, **caractérisée en ce que** l'unité (7) de régulation de la température est pourvue d'au moins un ventilateur (25).

10. Chambre de marquage par des isotopes selon l'une quelconque des revendications 1 à 9, **caractérisée en ce que** l'unité (8) d'humidification de l'air comprend un transducteur à ultrasons (32) piézocéramique.

11. Chambre de marquage par des isotopes selon l'une quelconque des revendications 1 à 10, **caractérisée en ce que** l'unité (9) d'absorption de gaz contient un matériau qui absorbe le dioxyde de carbone.

12. Chambre de marquage par des isotopes selon la revendication 11, **caractérisée en ce que** le matériau absorbant le dioxyde de carbone est la chaux sodée.

13. Chambre de marquage par des isotopes selon l'une quelconque des revendications 1 à 12, **caractérisée en ce que** le sas (6) comprend au moins deux bandes (20) élastiques, se chevauchant de manière croisée ou en étoile.

14. Procédé pour le marquage de plantes par des isotopes stables **caractérisé par** l'utilisation de la chambre de marquage par des isotopes selon l'une quelconque des revendications 1 à 13.

15. Procédé selon la revendication 14, **caractérisé en ce qu'**il s'agit, pour les plantes, de maïs, de riz, de blé, de soja ou de colza.
